# FASCICULE DE BREVET EUROPEEN

(11) **EP 1 844 786 B1**
(45) Date de publication et mention de la délivrance du brevet: **13.10.2010**
(21) Numéro de dépôt: 07368005.0
(22) Date de dépôt: 02.04.2007
(51) Int. Cl.: A61K 36/9066, A61K 47/00, A61Q 19/00, A61P 17/08, A61P 17/10, A61P 17/14

(54) **Extrait de curcuma longa et ses applications cosmétiques ou dermopharmaceutiques**
Curcuma longa Extrakte und deren kosmetischen und dermatologischen Anwendungen
Extracts of Curcuma longa and their cosmetic and dermatological uses

(30) Priorité: 03.04.2006 FR 0602879
(43) Date de publication de la demande: 17.10.2007
(73) Titulaire: EXSYMOL S.A.M., 98000 Monaco (MC)
(72) Inventeur: Seguin, Marie-Christine, 98000 Monaco (MC)
(74) Mandataire: Macquet, Christophe

(56) Documents cités:
- US-A- 5 897 865
- DATABASE WPI Week 200432 Derwent Publications Ltd., London, GB; AN 2004-344772 XP002414748 "External preparation for skin" & JP 2004 131498 A (LIFE CREATE KK) 30 avril 2004 (2004-04-30)
- DATABASE WPI Week 200562 Derwent Publications Ltd., London, GB; AN 2005-601083 XP002414749 & JP 2005 239697 A (NOZAKI K) 8 septembre 2005 (2005-09-08)
- PUROHIT A. ET AL.: "Contraceptive effect of Curcuma longa in male albinos rats." ASIAN J. ANDROL., vol. 6, mars 2004 (2004-03), pages 71-74, XP002414744
- DORAI T. ET AL.: "Therapeutic potential of curcumin in human prostate cancer- Curcumin induces apoptosis in both androgen-dependent and andogen-independent prostate cancer cells." PROSTATE CANCER AND PROSTATIC DISEASES, vol. 3, 2000, pages 84-93, XP002414745
- GREENLEE HEATHER ET AL: "SEPARATE EFFECTS OF NATUROPATHIC DIETARY CHANGES AND A COMBINATION BOTANICAL SUPPLEMENT ON SERUM SEX STEROID HORMONES IN PREMENOPAUSAL WOMEN" CANCER EPIDEMIOLOGY, BIOMARKERS AND PREVENTION, AMERICAN ASSOCIATION FOR CANCER RESEARCH,, US, vol. 14, no. 11 SUPS PART 2, 30 octobre 2005 (2005-10-30), page 2695S, XP009076724 ISSN: 1055-9965

## Description

L'invention concerne un extrait obtenu à partir des parties aériennes de *Curcuma longa,* une composition cosmétique ou dermopharmaceutique contenant un tel extrait, ainsi que l'utilisation d'un tel extrait ou d'une telle composition pour réguler ou inhiber des mécanismes androgéno-dépendants au niveau de la peau ou du cuir chevelu.

Nombreuses sont les personnes qui, de nos jours, sont affectées, à divers degrés, par un ou plusieurs désordres dits androgéno-dépendants, ayant comme origine commune un dérèglement du métabolisme de l'hormone androgène testostérone (P. Vexiau, « Hormones et peau », Cosmétologie (1996), N° 10, pp. 43-47). Ces dérèglements peuvent être d'origine génétique ou pathologique, mais peuvent aussi être liés au processus de vieillissement. Au niveau de la peau ou du cuir chevelu, les plus courants de ces dysfonctionnements hormonaux sont la pousse de poils non désirés sur le corps ou sur le visage, avec notamment l'hirsutisme chez la femme, l'acné, l'hyperséborrhée, ou l'alopécie.

Un autre constat est que, de nos jours, dans les sociétés occidentales, les hommes, presque autant que les femmes, cherchent à réduire leur pilosité. Chez les deux sexes, une peau glabre est en effet devenue symbole de beauté. Pour répondre à cette aspiration croissante, de nouvelles techniques d'épilation se sont développées. Toutefois, elles sont le plus souvent contraignantes et coûteuses. Une alternative consiste aujourd'hui à appliquer sur la peau des compositions pouvant interférer localement avec le métabolisme de la testostérone, le principal régulateur de la pousse du poil.

D'une manière générale, il existe une réelle demande quant à fournir des produits ou préparations permettant d'agir sur les mécanismes androgéno-dépendants, tant au niveau de la peau que du cuir chevelu.

Ainsi, dans l'optique de satisfaire ce besoin, la demanderesse a découvert, curieusement, de manière inattendue, et c'est le fondement de la présente invention, que des extraits issus de feuilles ou fleurs de *Curcuma longa,* présentaient une remarquable activité de réduction ou d'inhibition sur la métabolisation de la testostérone, procurant ainsi une nouvelle réponse pour le traitement de désordres androgéno-dépendants, pour des besoins esthétiques et dermatologiques.

Parmi les formulations présentes sur le marché pour lesquelles il est affirmé une action générale "anti-androgène", utile pour les applications susmentionnées, il est à relever dans l'état de la technique la présence de nombreuses préparations ou associations d'origine végétale. Ainsi, à titre d'exemple, il peut être cité un extrait éthanolique des plantes Majihto et/ou Kachur préconisé pour lutter contre l'alopécie, la séborrhée et l'acné (brevet JP2002/241297). Une composition cosmétique ou dermatologique à base d'un extrait de myrrhe (*Commiphora myrrha*) est destinée à réguler la sécrétion sébacée des peaux à tendance grasse ou acnéique, mais aussi à traiter l'acné, l'hirsutisme, ainsi qu'à ralentir la pousse de poils sur le visage (brevet WO00/04872). Une autre composition d'application topique, à base notamment d'un extrait de bois de cédar et de canelle, trouve une application dans le contrôle de la séborrhée et dans le traitement de l'acné (brevet US2004/228822). Il est attribué à des substances extraites de plantes japonaises une action d'inhibition des récepteurs androgéniques, dont l'intérêt s'exercerait tout particulièrement vis à vis de l'acné et de l'alopécie masculine (brevets JP2000/001432 et JP8193094). Pour certaines de ces préparations caractérisées par une présence végétale, il est parfois fait état, clairement, tel dans la demande internationale WO02/19974, d'un effet inhibiteur sur l'enzyme 5-alpha réductase, enzyme d'intérêt puisque responsable de la conversion de la testostérone en un autre androgène plus puissant et particulièrement actif sur les glandes sébacées de la peau et sur le follicule pileux, la dihydroxytestostérone.

La plante herbacée *Curcuma longa,* de la famille botanique des Zingibéracées, a elle aussi été citée. A la lecture de la monographie de cette plante (Alternative Medicine Review (2001), vol.6, pp. S62-S66) et des exemples ci-après de préparations portés à la connaissance du public, il s'avère que c'est le rhizome, à savoir la tige souterraine, qui constitue la partie de la plante utilisée classiquement à des fins médicales, et notamment pour le traitement de désordres androgéno-dépendants. Le rhizome, une fois bouilli, lavé et séché, donne une poudre jaune (ou "turmeric" en anglais) riche de composés phénoliques appelés curcuminoïdes ("Le curcuma", Nutranews, Juin 2002), telles la curcumine, la tétrahydrocurcumine, la diméthoxycurcumine, etc. Et il est attribué à cette poudre jaune des propriétés antioxydante, hépatoprotectrice, anti-inflammatoire, anticarcinogène et antimicrobienne, qui font d'ailleurs l'intérêt du *Curcuma longa* en médecines traditionnelle et ayurvédique ("La médecine ayurvédique", Nutranews, Septembre 2003). Ainsi, à titre d'exemples impliquant le rhizome de curcuma en tant que tel ou indirectement ce dernier dès lors qu'il est mis en exergue la curcumine ou un analogue de celle-ci, il est relevé les demandes de brevets ou brevets JP3176414, WO03/088927, JP9255568 et US2003/105030. Le premier document mentionne que l'acné et l'alopécie sont les cibles d'un agent cosmétique à base de plantes japonaises et chinoises, notamment à base de rhizome de curcuma. Les trois suivants divulguent l'utilisation de la curcumine ou ses analogues pour s'opposer à divers troubles androgéno-dépendants.

Malgré ces nombreux enseignements, il est à constater qu'il n'a jamais été rapporté, ni même suggéré, un effet réducteur ou inhibiteur sur la métabolisation de la testostérone pour des extraits de *Curcuma longa* lorsque ceux-ci résultent exclusivement des parties aériennes de la plante, pas plus qu'il n'a été décrit l'utilisation de ce même type d'extrait, ou compositions à base de celui-ci, à des fins de lutte contre la pousse de poils non désirés sur le visage ou sur le corps, de prévention ou de lutte contre l'acné, l'hyperséborrhée et l'alopécie séborrhéique masculine.

Avec l'expression "parties aériennes" précédemment citée, il est désigné les fleurs et les feuilles de la plante. Les feuilles et les fleurs de *Curcuma longa* ont certes été l'objet de recherches, la plupart concernant cependant quasi-systématiquement des huiles essentielles obtenues par hydrodistillation. La littérature révèle, pour ces huiles volatiles de feuilles ou de fleurs, une large proportion en dérivés monoterpéniques, notamment en terpinolènes (Chane-Ming J. et al., J. Essent. Oil Res. (2002), vol.14, pp.249-251). Il est notamment divulgué, pour une huile essentielle à base de feuilles de curcuma, une activité anti-inflammatoire (Lyengar M.A. et al., Indian Drugs (1994), vol.31, pp.528-531) ainsi qu'une action insecticide vis à vis de coléoptères (Tripathi A.K. et al., J. Econ. Entomol. (2002), vol.95, pp.183-189).

Selon donc un premier aspect, l'invention a pour objet un extrait de *Curcuma longa* :
- qui est obtenu à partir des parties aériennes de la plante, parties récoltées à une période précise de l'année pour garantir des teneurs maximales en composés actifs, de préférence en automne (mois de Mars et Avril) lorsque récoltées à une latitude proche de celle du tropique du Capricorne,
- caractérisé en ce qu'il présente une activité de réduction ou d'inhibition sur la métabolisation de la testostérone.
L'extrait de *Curcuma longa* peut être avantageusement isolé à partir des parties aériennes de la plante, déshydratées et broyées.

Une autre caractéristique dudit extrait est qu'il contient notamment des dérivés de type phénylpropanoïde et des dérivés de type flavonoïde, substances qui sont de plus utilisées comme des traçeurs permettant de "standardiser" l'extrait. Il est important de souligner, par contre, l'absence totale de curcumine et de composés apparentés dans les parties aériennes de la plante. Les propriétés affichées par un extrait obtenu à partir de ces parties aériennes ne sont donc pas imputables à la présence de telles substances, contrairement au rhizome de *Curcuma longa.*

Cet extrait, qui constitue en lui-même un produit nouveau, contient des dérivés de type phénylpropanoïde et flavonoïde, dans une teneur massique comprise entre 0,05 et 3 %.

Ledit extrait peut être obtenu selon une technique bien connue de l'homme de l'art. Tout d'abord, les parties aériennes de *Curcuma longa* sont récoltées, déshydratées et grossièrement broyées. Une extraction est ensuite effectuée à l'aide de solvants alcooliques, hydroalcooliques ou tout autre solvant polaire (DMF, DMSO, acétonitrile, etc), sous agitation et à reflux. Une fois l'extraction réalisée, et après épuisement du végétal, les solvants organiques sont éliminés, puis le volume d'extrait est ajusté avec de l'eau. Il est aussi possible de rajouter un glycol miscible à l'eau pour garantir la solubilité de certains constituants de l'extrait.

Selon une variante avantageuse, dans un extrait dosé à 0,1% en poids de dérivés flavonoïdes, l'extrait contient de 0,01 à 0,5 % en poids de dérivés de type phénylpropanoïde, préférentiellement de 0,01 à 0,1 %.

Selon un mode de réalisation avantageux de l'invention, afin d'obtenir un extrait plus actif et plus stable, une réaction d'hydrolyse acide ménagée est conduite sur l'extrait en solution, permettant notamment l'obtention de dérivés aglycones de type flavonoïde. L'activité renforcée de cet extrait peut s'expliquer en partie par la formation de composés pénétrant plus facilement dans l'épiderme, tels les flavonoïdes aglycones.

Les dérivés aglycones de type flavonoïde qui ont pu être identifiés dans l'extrait et qui sont utilisés comme traceurs pour standardiser l'extrait sont la quercétine et le kaempférol, alors que les dérivés de type phénylpropanoïde identifiés sont l'acide férulique et l'acide coumarique.

Selon un deuxième aspect, la présente invention s'étend également à une composition cosmétique ou dermopharmaceutique comprenant, à titre d'agent agent réducteur ou inhibiteur de la métabolisation de la testostérone, une quantité efficace de l'extrait tel que défini précédemment. Celui-ci est incorporé dans la composition avec tout excipient cosmétiquement ou pharmaceutiquement acceptable. Une telle composition est substantiellement exempte d'extrait de rhizome de *Curcuma longa.*

Avantageusement, la teneur en extrait dans la composition ci-dessus est comprise entre 1 et 10 % en poids par rapport au poids total de la composition, de préférence entre 1 et 5 % en poids.

Ladite composition est susceptible de comprendre, en outre, un autre élément actif choisi parmi des agents pour lesquels il est reconnu un effet inhibiteur sur l'activité d'enzymes spécifiques, dont on sait qu'elles sont impliquées dans le métabolisme de la testostérone au niveau de la peau ou du cuir chevelu. Ces enzymes peuvent être la protéine kinase C, l'ornithine décarboxylase, l'adenylosuccinate synthetase, l'aspartate transcarbamylase, la gamma-glutamyl transpeptidase, la S-adenosylmethionine decarboxylase, la NO-synthétase (ou NO-synthase), la sérine protéase, et la 5-alpha-reductase. Selon un mode préféré de réalisation de la présente invention, il est sélectionné un agent capable d'inhiber l'activité de l'enzyme ornithine décarboxylase, tel le chlorhydrate d'efflornithine, ou celle de l'enzyme NO-synthase, tel le N^{G}-nitro-L-arginine methylester.

Selon un mode tout particulier de l'invention visant un bénéfice esthétique associé à une limitation de la repousse des poils, ladite composition peut comprendre, toujours de manière facultative, également de l'urée (jusqu'à une concentration de 20 %) et le N^{G}-nitro-L-arginine methylester.

Les compositions cosmétiques ou dermopharmaceutiques sont formulées pour être appliquées topiquement sur la peau sous forme, par exemple, de lait, gel, crème, lotion, émulsion, etc. Elles peuvent être aussi administrées par voie orale (administration per os) sous forme de comprimés, gélules, capsules, ampoules, sirop, gouttes, etc.

A titre d'illustration, il est mentionné ci-après quelques exemples de formulation de composition cosmétique ou dermopharmaceutique selon l'invention, contenant l'extrait de *Curcuma longa* précité :

**Formule A (application topique : anti-repousse-poil)**

| | |
|---|---|
| Curcuma extract (extrait de curcuma) | 5 % |
| Hydrogenated polydecene (polydécène hydrogéné) | 5 % |
| Caprilic/Capric Triglycerides (triglycérides capriques/capriliques) | 2 % |
| Ethoxydiglycol oleate (oléate d'éthoxydiglycol) | 8 % |
| Glyceryl stearate (stéarate de glycéryle) | 3 % |
| Dimethicone (diméthicone) | 1 % |
| PEG-100 stearate (and) glyceryl stearate (stéarate de polyéthylène glycol-100 et stéarate de glycéryle) | 5 % |
| Glycerin (glycérine) | 3 % |
| Carbomer (copolymère d'acrylate/acrylamide & huile minérale) | 0,2 % |
| Sodium hydroxide (soude) | 0,4 % |
| Chlorphenesin (chlorphénésine) | 0,2 % |
| Phenonip (parabènes (parahydroxybenzoate) de butyle, éthyle, isobutyle, méthyle, propyle et phénoxyéthanol) | 0,6% |
| Wheat germ oil (huile de germe de blé) | 1,5 % |
| Sesamus indicum oil (huile de sésame) | 1,5 % |
| PEG-8 & Tocopherol & Ascorbyl Palmitate & Ascorbic acid & Citric acid (polyéthylène glycol-8 & tocophérol & palmitate d'ascorbyle & acide ascorbique & acide citrique) | 0,07 % |
| Urea (urée) | 5 % |
| Efflornithine chlorhydrate (chlorhydrate d' efflornithine) | 1 % |
| NG-nitro-L-arginine methyl ester (ester méthylique du NG-nitro-L-arginine) | 1 % |
| Aqua (eau) | qsp 100 % |

**Formule B (application topique : alopécie séborrhéique masculine)**

| | |
|---|---|
| Curcuma extract (extrait de curcuma) | 3 % |
| Glyceryl stearate & stearyl alcohol (stéarate de glycéryle & alcool stéarylique) | 5 % |
| Stearyl alcohol (alcool stéarylique) | 2 % |
| Hydrogenated polydecene (polydécène hydrogéné) | 8 % |
| Caprilic/Capric Triglyceride (triglycérides capriques/capriliques) | 5 % |
| Cetearyl isononanoate (isononanoate de cétéaryle) | 2 % |
| Dicaprylyl carbonate (carbonate de dicaprylylique) | 1 % |
| Cera alba (cire d'abeille) | 1 % |
| Propylparaben (parabène de propyle) | 0,3 % |
| Aqua (eau) | 10 % |
| Ethylenediamine-tetraacetic acid disodium salt dihydrate (EDTA) (éthylènediamine-sel disodique dihydraté de l'acide tétraacétique) | 0,2 % |
| Glycerin (glycérine) | 3 % |
| Xantha gum (gomme xanthique) | 0,4 % |
| Emulzome Macadamia (polyisobutène hydrogéné & hepatanoate de stéaryle & huile de macadamia & parabène de méthyle) | 5 % |
| Triethanolamine (triéthanolamine) | 0,35 % |
| Aqua (eau) | qsp 100 % |

**Formule C (application topique : acné)**

| | |
|---|---|
| Curcuma extract (extrait de curcuma) | 2 % |
| Hydrogenated polydecene (polyisobutène hydrogéné) | 8 % |
| Caprilic/Capric Triglyceride (triglycérides capriques/capriliques) | 2 % |
| Ethoxydiglycol Oleate (oléate d'éthoxydiglycol) | 8 % |
| Glyceryl stearate (stéarate de glycéryle) | 2 % |
| Dimethicone (diméthicone) | 1 % |
| PEG-100 stearate (and) glyceryl stearate (stéarate de polyéthylène glycol-100 et stéarate de glycéryle) | 5 % |
| Propylparaben (parabène de propyle) | 0,3 % |
| Stearyl alcohol (alcool stéarylique) | 1 % |
| Aqua (eau) | 10 % |
| Ethylenediamine-tetraacetic acid disodium salt dihydrate (EDTA) (éthylènediamine-sel disodique dihydraté de l'acide tétraacétique) | 0,2 % |
| Glycerin (glycérine) | 2 % |
| Xantha gum (gomme xanthique) | 0,4 % |
| Wheat germ oil (huile de germe de blé) | 1 % |
| Macadamia Ternifolia seed oil (huile de graines de Macadamia) | 1 % |
| PEG-8 & Tocopherol & Ascorbyl Palmitate & Ascorbic acid & Citric acid (polyéthylène glycol-8 & tocophérol & palmitate d'ascorbyle & acide ascorbique & acide citrique) | 0,07% |
| Triethanolamine (triéthanolamine) | 0,35 % |
| Aqua (eau) | qsp 100 |

**Formule D (application : anti-repousse-poil, administration per os)**

| | |
|---|---|
| Curcuma extract (extrait de curcuma) | 4 % |

Excipients qsp 1 capsule en gelatine : lécithine de soja, huile de soja, glycérides d'acides gras

Selon un troisième aspect, la présente invention concerne l'utilisation d'un extrait de *Curcuma longa* tel que défini précédemment comme agent cosmétique destiné à réduire ou inhiber la métabolisation de la testostérone, en particulier pour ralentir la pousse des poils non désirés du visage ou du corps, ou pour lutter contre ou prévenir l'hyperséborrhée et l'alopécie masculine. Préférentiellement, il est cherché, avec ledit extrait, à ralentir la pousse de poils non désirés du visage ou du corps.

Selon un autre aspect de l'invention, la présente invention concerne l'utilisation d'un extrait de *Curcuma longa* tel que défini précédemment pour la fabrication d'une composition dermopharmaceutique destinée à réduire ou inhiber la métabolisation de la testostérone, en particulier pour lutter contre ou prévenir l'acné et l'hirsutisme. Elle concerne aussi l'utilisation d'un extrait de *Curcuma longa* tel que défini précédemment pour la fabrication d'une composition cosmétique destinée à réduire ou inhiber la métabolisation de la testostérone, en particulier pour ralentir la pousse des poils non désirés du visage ou du corps, ou pour lutter contre ou prévenir l'hyperséborrhée et l'alopécie androgéno-dépendante. Préférentiellement, il est cherché, avec cette dernière composition, à ralentir la pousse des poils non désirés du visage ou du corps.

Enfin, l'invention est illustrée, à titre purement indicatif, d'une part, par le test *in vitro* ci-après (test 1).

### Test 1 : mise en évidence de l'effet d'un extrait de Curcuma longa sur la métabolisation de la testostérone dans des épidermes reconstruits d'origine humaine (fournisseur : SkinEthic^{®}).

Ces épidermes constituent un matériel biologique présentant une forte homologie avec l'épiderme humain, et permettant de tester de manière réaliste un produit destiné à être appliqué à la surface de la peau.
Un extrait glycolique de feuilles de *Curcuma longa* a donc été testé, à différentes concentrations. Leur action a par ailleurs été comparée à celle d'un inhibiteur spécifique de la 5-alpha-réductase, le Finastéride^{®} (molécule de référence dans ce type d'évaluation).
Des épidermes reconstruits de 17 jours ont ainsi été pré-cultivés en plaque 24 puits, pendant 24 heures. Puis 100 µl de testostérone marquée ont été déposés sur la face cornée de chaque épiderme. Après 24 nouvelles heures, les milieux de culture ont été prélevés pour analyse. Il est alors regardé la métabolisation de la testostérone par chromatographie sur plaque de silice et par comptage direct de la radioactivité des différents spots, à l'aide du logiciel "Optiquant version 3 (packard)". Différents métabolites, et en particulier la dihydrotestostérone (DHT), sont identifiés à l'aide de standards. Chaque point est la moyenne de 3 mesures effectuées sur des épidermes différents.

Les résultats sont rassemblés dans le tableau suivant :

| | % inhibition métabolisation testostérone | % inhibition transformation en DHT | Ratio DHT/testo. |
|---|---|---|---|
| Témoin non traité | - | - | 2,2 |
| Finastéride 10⁻⁴M | 54 | 92 | 0,1 |
| Extrait glycolique 0,1% | 38 | 4 | 1,5 |
| Extrait glycolique 0,5% | 70 | 40 | 0,5 |
| Extrait glycolique 1% | 85 | 51 | 0,2 |

L'extrait glycolique montre une forte activité anti-androgène révélée par une inhibition dose-dépendante de la métabolisation de la testostérone (le % d'inhibition est exprimé par rapport au témoin non traité). La comparaison avec le Finastéride, un inhibiteur spécifique de l'enzyme 5-alpha reductase, montre que l'extrait inhibe cette enzyme, mais agit aussi sur d'autres enzymes impliqués dans la métabolisation de la testostérone (effet révélé par la chromatographie).

D'autre part, toujours à titre indicatif, l'invention est également illustrée par les trois tests *in vivo* ci-après (tests 2 à 4).

### Test 2 : mise en évidence de l'effet, par voie topique, d'une composition cosmétique précitée sur la pousse de poils non désirés.

Une étude statistique a été conduite sur un panel de 25 hommes et femmes de 20 à 50 ans, à la pousse particulièrement vive de poils sous les aisselles. Il a été procédé à un tirage au sort pour connaître les sujets "traités" et les sujets "non traités". Parmi les sujets "traités" figuraient des hommes et femmes à s'appliquer, après rasage des aisselles, quotidiennement, matin et soir, pendant 20 jours, ou la formule A précitée sous forme de lotion, ou une lotion "placebo" (sans substance active).
Les résultats sont rassemblés dans le tableau suivant :

| | **% de sujets avec réduction longueur de poil** |
|---|---|
| 10 sujets "traités" : formule A | 80% |
| 7 sujets "traités" : placebo | 14 % |
| 8 sujets "non traités" | 0 % |

Après seulement 20 jours de traitement, il est observé, en comparaison à des sujets qui n'ont reçu aucun traitement, que 80 % des sujets "traités" qui ont appliqué la formule A objet de la présente invention ont vu la longueur de leurs poils sous l'aisselle réduite d'au moins 39 %.

### Test 3 : mise en évidence de l'effet, par voie topique, d'un extrait de Curcuma longa vis-à-vis de l'alopécie séborrhéique masculine.

Dans le cadre d'une étude préliminaire, un extrait glycolique de feuilles de *Curcuma longa,* dosé à 0,25 % en actifs, a été testé sur huit hommes de moins de 40 ans présentant des signes précoces d'alopécie de type androgénétique. Il est à signaler que l'activité d'un extrait/formulation, à des fins de lutte contre la chute des cheveux, peut être évaluée, en première intention, en suivant le rapport du nombre de cheveux en phase anagène sur le nombre de cheveux en phase télogène. Ainsi, un rapport inférieur à 4 dénote une perturbation suspecte pouvant être classée comme pathologique si elle devient inférieure à 3 (P. Bouhanna, "Garder et retrouver ses cheveux", Ed. Springer-Verlag, 2000, et références citées).

Cette étude de pré-évaluation clinique a donc été réalisée pendant 3 mois sur ces huit volontaires, à raison de deux applications par jour, matin et soir. Il est noté les résultats suivants (confirmant le potentiel anti-alopécie de cet actif et de résultats très positifs dans une formulation développée) :
- cinq cas ont vu leur rapport initial "cheveux en phase anagène sur cheveux en phase télogène" sensiblement augmenté, et devenir nettement supérieur à 5, avec l'observation d'un recouvrement partiel visible de quelques zones du cuir chevelu dénudées
- 1 seul cas n'a présenté aucune amélioration. A noter que deux cas n'ont pas été classés comme "positif", malgré un rapport augmenté et une amélioration visible de la qualité de la pousse des cheveux.

### Test 4 : mise en évidence de l'effet d'une composition précitée, administrée par voie orale, sur la pousse de poils non désirés.

Parmi des femmes fréquentant un Institut de Beauté, il a été sélectionné 12 femmes volontaires, aux visites régulières (une fois par mois) dans cet Institut pour subir une épilation à la cire au niveau des jambes. Dans le cadre du présent test, ces femmes ont accepté d'absorber, par vois orale sous forme de capsules, un extrait huileux de *Curcuma longa* (formule D précitée). A raison d'une capsule le matin à jeun, la durée du traitement a été d'un mois. Au terme de ce dernier, une enquête réalisée auprès de ces 12 femmes a démontré, pour chacune d'entre elles, un retard sur la date d'épilation "nécessaire" dans ledit Institut, justifiant ainsi d'un effet significatif anti-repousse poil. Il a été ainsi observé :
- que, pour 5 d'entre elles, le retard a été de 1 semaine environ
- que, pour 4 d'entre elles, le retard a été de 2 semaines
- que, pour les 3 restantes, le retard a été tel qu'elles ont pu éviter une séance mensuelle

## Revendications

1. Extrait de *Curcuma longa* obtenu à partir des parties aériennes de la plante, **caractérisé en ce qu'**il contient des dérivés de type phénylpropanoïde et des dérivés de type flavonoïde.

2. Extrait selon la revendication 1, **caractérisé en ce qu'**il est obtenu à partir des parties aériennes de la plante, déshydratées et broyées.

3. Extrait selon l'une des revendications 1 et 2, **caractérisé en ce qu'**il contient des dérivés de type phénylpropanoïde et flavonoïde, dans une teneur massique comprise entre 0,05 et 3 %.

4. Extrait selon l'une des revendications 1 à 3, **caractérisé en ce qu'**il contient, dans un extrait dosé à 0,1% en poids de dérivés flavonoïdes, de 0,01 à 0,5 % en poids de dérivés de type phénylpropanoïde.

5. Extrait selon la revendication 4, **caractérisé en ce qu'**il contient de 0,01 à 0,1 % en poids de dérivés de type phénylpropanoïde.

6. Extrait selon l'une des revendications 1 à 5, **caractérisé en ce que** les dérivés aglycones de type phénylpropanoïde sont choisis dans le groupe constitué par l'acide férulique et l'acide coumarique et **en ce que** les dérivés de type flavonoïde sont choisis dans le groupe constitué par le kaempférol et la quercétine.

7. Composition cosmétique ou dermopharmaceutique, **caractérisée en ce qu'**elle comporte, à titre d'agent réducteur ou inhibiteur de la métabolisation de la testostérone, une quantité efficace d'un extrait de *Curcuma longa* tel que défini à l'une des revendications 1 à 6, et un excipient cosmétiquement ou pharmaceutiquement acceptable.

8. Composition selon la revendication 7, **caractérisée en ce que** la teneur en extrait est comprise entre 1 et 10 % en poids par rapport au poids total de la composition.

9. Composition selon la revendication 8, **caractérisée en ce que** la teneur en extrait est comprise entre 1 et 5 % en poids par rapport au poids total de la composition.

10. Composition selon l'une des revendications 7 à 9, **caractérisée en ce qu'**elle comprend, en outre, un autre élément actif choisi parmi les agents inhibiteurs des enzymes protéine kinase C, ornithine décarboxylase, adenylosuccinate synthetase, aspartate transcarbamylase, gamma-glutamyl transpeptidase, S-adenosylmethionine decarboxylase, NO-synthétase, sérine protéase et 5-alpha-reductase.

11. Composition selon l'une des revendications 7 à 10, **caractérisée en ce qu'**elle comprend en outre de l'urée et le N^{G}-nitro-L-arginine methylester.

12. Composition selon l'une des revendications 7 à 11, **caractérisée en ce qu'**elle est formulée pour être appliquée topiquement sur la peau sous forme de lait, gel, crème, lotion, émulsion.

13. Composition selon l'une des revendications 7 à 11, **caractérisée en ce qu'**elle est formulée pour être administrée par voie orale sous forme de comprimés, gélules, capsules, ampoules, sirops, gouttes.

14. Utilisation d'un extrait de *Curcuma longa* selon l'une des revendications 1 à 6, comme agent cosmétique destiné à réduire ou inhiber la métabolisation de la testostérone, pour ralentir la pousse des poils non désirés du visage ou du corps, ou pour lutter contre ou prévenir l'hyperséborrhée et l'alopécie masculine.

15. Utilisation selon la revendication 14, dans laquelle l'extrait est utilisé pour ralentir la pousse des poils non désirés du visage ou du corps.

16. Utilisation d'un extrait de *Curcuma longa* selon l'une des revendications 1 à 6, pour la fabrication d'une composition dermopharmaceutique destinée à lutter contre ou prévenir l'acné et l'hirsutisme.

17. Utilisation d'une composition cosmétique contenant un extrait de *Curcuma longa* selon l'une des revendications 1 à 6, pour ralentir la pousse des poils non désirés du visage ou du corps, ou pour lutter contre ou prévenir l'hyperséborrhée et l'alopécie androgéno-dépendante.

18. Utilisation selon la revendication 17, dans laquelle la composition est utilisée pour ralentir la pousse des poils non désirés du visage ou du corps.

## Claims

1. Extract of *Curcuma longa* obtained from the aerial parts of the plant, **characterized in that** it contains derivatives of phenylpropanoid type and derivatives of flavonoid type.

2. Extract according to claim 1, **characterized in that** it is obtained from dehydrated and crushed aerial parts of the plant.

3. Extract according to one of claims 1 and 2, **characterized in that** it contains derivatives of phenylpropanoid type and derivatives of flavonoid type, in a weight content comprised between 0.05 and 3%.

4. Extract according to one of claims 1 to 3, **characterized in that** it contains from 0.01 to 0.5% in weight of derivatives of phenylpropanoid type, in a titrated extract with 0.1% in weight of flavonoid derivatives.

5. Extract according to claim 4, **characterized in that** it contains from 0.01 to 0.1% in weight of derivatives of phenylpropanoid type.

6. Extract according to one of claims 1 to 5, **characterized in that** aglycone derivatives of phenylpropanoid type are chosen in the group constituted of ferulic acid and coumaric acid, and **in that** derivatives of flavonoid type are chosen in the group constituted of kaempferol and quercetin.

7. Cosmetic or dermopharmaceutical composition, **characterized in that** it comprises, as a reducing or inhibitor agent of the testosterone metabolization, an effective amount of an extract of *Curcuma longa* such as defined to one of claims 1 to 6, and a cosmetically or pharmaceutically acceptable excipient.

8. Composition according to claim 7, **characterized in that** the content in extract is comprised between 1 and 10% in weight in relation to total weight of the composition.

9. Composition according to claim 8, **characterized in that** the content in extract is comprised between 1 and 5% in weight in relation to total weight of the composition.

10. Composition according to one of claims 7 to 9, **characterized in that** it comprises furthermore another active element chosen among the inhibitor agents of protein kinase C, ornithine decarboxylase, adenylosuccinate synthetase, aspartate transcarbamylase, gamma-glutamyl transpeptidase, S-adenosylmethionine decarboxylase, NO-synthetase, serine protease and 5-alpha-reductase enzymes.

11. Composition according to one of claims 7 to 10, **characterized in that** it comprises furthermore urea and N^{G}-nitro-L-arginine methylester.

12. Composition according to one of claims 7 to 11, **characterized in that** it is formulated to be topically applied on the skin under the form of milk, gel, cream, lotion, emulsion.

13. Composition according to one of claims 7 to 11, **characterized in that** it is formulated to be administered by oral route under the form of tablets, hard capsules, capsules, ampules, syrups, drops.

14. Use of an extract of *Curcuma longa* according to one of claims 1 to 6, as a cosmetic agent intended for reducing or inhibiting the testosterone metabolization, for slowing down the unwanted body hair or face air growth, or for fighting against or preventing hyperseborrhea and male alopecia.

15. Use according to claim 14, in which the extract is used to slow down the unwanted body hair or face air growth.

16. Use of an extract of *Curcuma longa* according to one of *claims* 1 to 6, for the manufacture of a dermopharmaceutical composition intended for fighting against or preventing acne and hirsutism.

17. Use of a cosmetic composition consisting of an extract of *Curcuma longa* according to one of claims 1 to 6, for slowing down the unwanted body hair or face air growth, or for fighting against or preventing hyperseborrhea and androgeno-dependent alopecia.

18. Use according to claim 17, in which the composition is used for slowing down the unwanted body hair or face air growth.

## Patentansprüche

1. *Curcuma-longa*-Extrakt, der aus oberirdischen Teilen der Pflanze gewonnen wird, **dadurch gekennzeichnet, dass** er Derivate des Phenylpropanoid-Typs und Derivate des Flavonoid-Typs enthält.

2. Extrakt nach Anspruch 1, **dadurch gekennzeichnet, dass** er aus oberirdischen Teilen der Pflanze gewonnen wird, die dehydratisiert und zerstoßen wurden.

3. Extrakt nach einem der Ansprüche 1 und 2, **dadurch gekennzeichnet, dass** er Derivate des Phenylpropanoid- und Flavonoid-Typs in einem Gewichtsanteil enthält, der zwischen 0,05 und 3% liegt.

4. Extrakt nach einem der Ansprüche 1 bis 3, **dadurch gekennzeichnet, dass** er in einem Extrakt, der auf 0,1 Gew.-% Flavonoidderivate bemessen ist, 0,01 bis 0,5 Gew.-% Derivate des Phenylpropanoid-Typs enthält.

5. Extrakt nach Anspruch 4, **dadurch gekennzeichnet, dass** er 0,01 bis 0,1 Gew.-% Derivate des Phenylpropanoid-Typs enthält.

6. Extrakt nach einem der Ansprüche 1 bis 5, **dadurch gekennzeichnet, dass** die Aglykonderivate des Phenylpropanoid-Typs aus der Gruppe bestehend aus Ferulasäure und Cumarinsäure ausgewählt sind und dass die Derivate des Flavonoid-Typs aus der Gruppe bestehend aus Kaempferol und Querzetin ausgewählt sind.

7. Kosmetische oder dermopharmazeutische Zusammensetzung, **dadurch gekennzeichnet, dass** sie eine wirksame Menge eines wie in einem der Ansprüche 1 bis 6 definierten *Curcuma-longa*-Extrakts als Mittel zur Verringerung oder Inhibierung der Metabolisierung von Testosteron und einen kosmetisch oder pharmazeutisch unbedenklichen Trägerstoff umfasst.

8. Zusammensetzung nach Anspruch 7, **dadurch gekennzeichnet, dass** der Anteil des Extrakts zwischen 1 und 10 Gew.-% liegt, bezogen auf das Gesamtgewicht der Zusammensetzung.

9. Zusammensetzung nach Anspruch 8, **dadurch gekennzeichnet, dass** der Anteil des Extrakts zwischen 1 und 5 Gew.-% liegt, bezogen auf das Gesamtgewicht der Zusammensetzung.

10. Zusammensetzung nach einem der Ansprüche 7 bis 9, **dadurch gekennzeichnet, dass** sie außerdem einen weiteren Wirkstoff umfasst, der aus den Inhibitoren von Proteinkinase-C-Enzymen, Ornithindecarboxylase, Adenylosuccinatsynthetase, Aspartattranscarbamylase, gamma-Glutamyltranspeptidase, S-Adenoxylmethionindecarboxylase, NO-Synthetase, Serinprotease und 5-alpha-Reduktase ausgewählt ist.

11. Zusammensetzung nach einem der Ansprüche 7 bis 10, **dadurch gekennzeichnet, dass** sie außerdem Harnstoff und N^{G}-Nitro-L-Argininmethylester umfasst.

12. Zusammensetzung nach einem der Ansprüche 7 bis 11, **dadurch gekennzeichnet, dass** sie dazu formuliert ist, topisch auf die Haut in Form einer Milch, eines Gels, einer Creme, einer Lotion, einer Emulsion aufgetragen zu werden.

13. Zusammensetzung nach einem der Ansprüche 7 bis 11, **dadurch gekennzeichnet, dass** sie dazu formuliert ist, oral in Form von Tabletten, Gelkapseln, Kapseln, Ampullen, Sirupen, Tropfen verabreicht zu werden.

14. Verwendung eines *Curcuma-longa*-Extrakts nach einem der Ansprüche 1 bis 6 als Kosmetikum, das dazu gedacht ist, die Metabolisierung von Testosteron zu verringern oder zu inhibieren, um unerwünschten Haarwuchs im Gesicht oder am Körper zu verzögern oder um Hyperseborrhoe und Alopezie des Mannes zu bekämpfen oder zu verhindern.

15. Verwendung nach Anspruch 14, wobei der Extrakt dazu verwendet wird, unerwünschten Haarwuchs im Gesicht oder am Körper zu verzögern.

16. Verwendung eines *Curcuma-longa*-Extrakts nach einem der Ansprüche 1 bis 6 zur Herstellung einer dermopharmazeutischen Zusammensetzung, die dazu gedacht ist, Akne und Hirsutismus zu bekämpfen oder zu verhindern.

17. Verwendung einer kosmetischen Zusammensetzung, die einen *Curcuma-longa*-Extrakt nach einem der Ansprüche 1 bis 6 enthält, zur Verzögerung von unerwünschtem Haarwuchs im Gesicht oder am Körper oder zu Bekämpfung oder Verhinderung von Hyperseborrhoe und androgenetischer Alopezie.

18. Verwendung nach Anspruch 17, wobei die Zusammensetzung dazu verwendet wird, unerwünschten Haarwuchs im Gesicht oder am Körper zu verzögern.
